(19) **Europäisches Patentamt**
**European Patent Office**
**Office européen des brevets**

(11) **EP 4 011 285 A1**

(12) **EUROPEAN PATENT APPLICATION**

(43) Date of publication:
**15.06.2022 Bulletin 2022/24**

(21) Application number: **20212775.9**

(22) Date of filing: **09.12.2020**

(51) International Patent Classification (IPC):
**A61B 5/024** (2006.01)        **A61B 5/11** (2006.01)
**A61B 5/16** (2006.01)

(52) Cooperative Patent Classification (CPC):
**A61B 5/165; A61B 5/024; A61B 5/1116**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PENNING DE VRIES, Hendricus Theodorus Gerardus**
**Maria**
**5656 AE Eindhoven (NL)**
• **GROEN, Rob**
**5656 AE Eindhoven (NL)**

(74) Representative: **Philips Intellectual Property & Standards**
**High Tech Campus 52**
**5656 AG Eindhoven (NL)**

(54) **STRESS DETECTION DEVICE, SYSTEM AND METHOD FOR DETECTING MENTAL STRESS OF A PERSON**

(57)    The present invention relates to a stress detection system, device and method for detecting mental stress of a person. The system (1, 2) comprises an activity sensor (20) configured to acquire activity information related to activity of the person, a heart rate sensor (30) configured to acquire heart rate information indicating or allowing to compute the current heart rate of the person, and a stress detection device (10, 40, 60) for detecting mental stress of a person based on the acquired activity information and the acquired heart rate information. Hereby, posture changes of the person are taken into account to adapt a computed basal heart rate component and/or the computation of the basal heart rate component if a posture change is detected.

FIG.1

EP 4 011 285 A1

**Description**

FIELD OF THE INVENTION

The present invention relates to a stress detection device, system and method for detecting mental stress of a person.

BACKGROUND OF THE INVENTION

In order to enable personalized programs for stress reduction, an individual's stress level should be measured and monitored. One way to measure stress is by questionnaires, which have the advantage that validated versions are available, and that contextual information can be obtained regarding e.g. (perceived) causes of stress, current coping strategies, and (perceived) effects of stress on daily life. However, disadvantages are that questionnaires offer subjective data, that results are dependent on how respondents are feeling at the time of taking the questionnaire, and that they are inconvenient for continuous monitoring. As such, it would be preferable to (also) have objective measurements of stress.

Stress has been shown to induce a variety of (measurable) physiologic responses, which may be categorized into short term and long term responses. While long term stress is the one that may have detrimental effects on health, short term stress monitoring is highly interesting for delivering personalized programs for stress reduction as well. It may provide users with insight in their stress patterns, and help them to change their daily behavior and thereby reduce or prevent long term stress.

There exist a number of physiological parameters that have been associated to stress in literature, in particular heart rate (HR), heart rate variability (HRV), blood pressure (BP) and cortisol levels. From these parameters currently, only HR can be continuously measured unobtrusively and accurately. In addition to stress, heart rate is affected by many other factors like physical activity, sleep, posture, circadian rhythm, temperature, dehydration, food, caffeine, nicotine, alcohol, etc.

Mental stress is a form of stress that occurs because of how events in one's external or internal environment are perceived, resulting in the psychological experience of distress and anxiety. Mental stress is often accompanied by physiological responses.

SUMMARY OF THE INVENTION

It is an object of the present invention to provide a device, system and method that can reliably detect and, preferably, quantify mental stress of a person.

In a first aspect of the present invention a stress detection device for detecting mental stress of a person is presented, the device comprising

an activity input configured to obtain activity information related to activity of the person;

a heart rate input configured to obtain heart rate information indicating or allowing to compute the current heart rate of the person;

a processing unit configured to

- detect a posture change of the person from the obtained activity information,
- compute a basal heart rate component from the obtained heart rate information under consideration of the result of the posture change detection,
- compute an activity heart rate component from the obtained activity information,
- compute a mental stress heart rate component by subtracting, from the current heart rate of the person, the computed activity heart rate component and the computed basal heart rate component, and

an output configured to output the computed mental stress information.

In a further aspect of the present invention a stress detection system for detecting mental stress of a person is presented, the system comprising:

- an activity sensor configured to acquire activity information related to activity of the person;
- a heart rate sensor configured to acquire heart rate information indicating or allowing to compute the current heart rate of the person; and
- a stress detection device as disclosed herein for detecting mental stress of a person based on the acquired activity information and the acquired heart rate information.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea that mental stress events are detectable by separating the instantaneous heart rate signal into fractions (or components) related to basal metabolic life functions and physical effort, and a fraction not related to both. It has been found that the latter fraction has a direct relation to mental stress events.

Further, it has been found that posture changes of the person cost little physical effort but have a significant impact on heart rate. For instance, a change from a still sitting position to a still standing position may lead to 5-10 BPM (beats per minute) increased heart rate. At the same time, such posture changes trigger only little variation on a motion signal, e.g. from an acceleration sensor, used for determining physical effort. For this reason, posture change detection is provided according to the present invention to improve the mental stress detection accuracy, i.e. the detection of posture changes is used as an additional input of the detection of mental stress and the computation of the mental stress information, such as the presence of a mental stress event and/or the level of mental stress of the person.

In particular, according to the present invention it is detected if the person changes the posture, which information is then considered in the computation of a basal heart rate component to take the effect of the posture change on the heart rate into account. Based on the knowledge of the various fractions (i.e. components) that contribute to the instantaneous (i.e. current) heart rate, a mental stress heart rate component is computed by subtracting, from the current heart rate of the person, the component representing activity and the component representing the basal heart rate, which may have been calculated differently or adapted before if a posture change has been detected. Mental stress information can then be computed from this mental stress heart rate component. In this way a heart rate increase or decrease because of a posture change can be taken into account in the detection of mental stress from the measured heart rate.

In an implementation an adaptive filter may be used to cancel out the 'activity contributions' from the heart rate signal. The adaptive filter also handles variations in the relation between activity count, physical effort, and heart rate increases due to physical effort. The adaptive filters uses a DC-free input signal for correct operation. Therefore, the basal heart rate component may be subtracted from the heart rate signal before it is processed in the adaptive filter. After subtraction of the basal heart rate component and cancellation of the activity components, the remaining heart rate increases due to mental stress in unit BPM.

There are different options for considering the result of the posture change detection, i.e., to take into account that a posture change happened and/or the kind and/or amount of posture change, in the computation of the basal heart rate. One of these options is to compute the basal heart rate component from the obtained heart rate component and adapting the computed basal heart rate component if a posture change is detected. Another (additional or alternative) option is to adapt the computation of the basal heart rate component if a posture change is detected, in particular by taking a different computation method and/or adapting one or more parameters used in the computation. Generally, if no or no significant posture change is detected, the computed basal heart rate and the computation of the basal heart rate component are not adapted (i.e. not changed compared to the basal heart rate and its computation used if no posture change were taken into account at all.

According to an embodiment the processing unit is configured to compute the basal heart rate component as lower envelope of a heart rate signal of the obtained heart rate information. Thus, in order to estimate the basal heart rate, the lower envelope of the heart rate signal may be tracked. In an embodiment, an average heart rate signal may be computed from the obtained heart rate information, and the lower envelope of the heart rate signal or the average heart rate signal may be computed as basal heart rate component.

According to another embodiment the processing unit is configured to adapt the computation of the basal heart rate component if a posture change is detected by increasing, during a time window after detection of a posture change, the speed by which the basal heart rate follows the heart rate signal. In this way heart rate changes caused by the posture change can be tracked very fast compared to the normal tracking (if no posture change happens) leading to a more accurate estimation of the mental stress heart rate contribution and thus a more accurate computation of the person's mental stress.

The processing unit may further be configured to compute the basal heart rate component by taking the difference between the current value of the heart rate signal and the current value of the basal heart rate, multiplying the difference with a multiplication constant and obtaining a new value for basal heart rate by integration of the result. The basal heart rate component can thus be easily and quickly computed.

In a more advanced embodiment the processing unit is configured to use a first multiplication constant if the current value of the basal heart rate is larger than the current value of the heart rate signal, a second multiplication constant if the current value of the basal heart rate is smaller than the current value of the heart rate signal, and a third multiplication constant if a posture detection is detected, wherein the second multiplication constant is smaller than the first multiplication constant and the first multiplication constant is smaller than the third multiplication constant. Thus, different multiplication constants may be applied for the basal heart rate rising or falling, i.e., multiplication constants are selected based on heart rate being greater or smaller than basal heart rate. Hereby, in case of falling basal heart rate a faster tracking can be enabled compared to a rising basal heart rate. Further, another (third) multiplication constant that is larger than the other multiplication constants is used in case of a posture change to enable an even faster tracking of the heart rate.

In another embodiment, the processing unit is configured to compute the basal heart rate component from the obtained heart rate component and to adapt the computed basal heart rate component by increasing the computed basal heart rate component if the posture changes from a sitting or lying posture to a standing posture and by decreasing the computed basal heart rate component if the posture changes from a standing posture to a sitting or lying posture. This is based on the idea that in case of standing up the heart rate generally increases and in case of sitting or lying down the heart rate generally decreases. The accuracy of the mental stress detection can thus finally be improved.

There are different ways to detect a posture change of the person. In one embodiment the processing unit is configured to detect a posture change of the person from the obtained activity information by detecting a change of a measured acceleration above an acceleration threshold. For instance, if the person stands up the acceleration will increase beyond 1g (9.81 m/s$^2$), i.e. an increased acceleration will be taken as an indication of a posture change.

In another embodiment a change of the orientation of a sensor that is configured to acquire the activity information may be detected and used as indication for a posture change. For instance, if a smartwatch including a motion sensor is used for acquiring the activity information, its orientation will change if the person stands up from a sitting or lying posture to a standing posture. In most cases, a standing person has his hand pointing down to earth while a sitting person has his arms mostly in horizontal orientation.

In another embodiment changes in an activity type metric indicating the type of activity of the person determined from the obtained activity information may be detected and used as indication for a posture change. This metric may e.g. take values like 'Rest', 'Running', 'Cycling', 'Walking', 'Other', and changes in this metric may be used as indication of posture changes.

The activity information may comprise one or more of activity count information, action type information and accelerometer information. For instance, an activity count metric may be used as a measure for motion and/or effort. Activity count represents an average of the amount of variation in the accelerometer signals during a certain period.

The processing unit may further be configured to compute the activity heart rate component from the obtained activity information by minimizing the correlation between the activity heart rate component and the mental stress heart rate component. For instance, the signal AHR-BHR is used as input signal for an adaptive filter. According to a model, this signal holds the sum of the activity heart rate component and the mental stress heart rate component. The adaptive filter constructs the physical heart rate component by filtering an activity signal included in the activity information, e.g. an activity count signal. The mental stress heart rate component can be calculated by subtraction of the activity heart rate component from the adaptive filter input. The filter coefficients are adapted in such a way that correlation between the mental stress heart rate component and the activity signal becomes minimized.

There are various embodiment for implementing the heart rate sensor which may include one or more of a photoplethysmography sensor, a pulse oximetry sensor, a body-mounted camera, a remote camera (e.g. at some meters distance), an ECG sensor, a wristband pressure sensor, and a wristband tension sensor. Smartwatches, that may make use of and implement the present invention, may e.g. use PPG sensors to measure heart rate, but depending on circumstances ECG sensors, camera images or other methods may be used instead or in addition.

For implementing the activity sensor there are various embodiments as well. It may include one or more of an accelerometer, a body-mounted camera, a remote camera (e.g. at some meters distance), an electrodermal activity sensor, a gyrometer, and a temperature sensor. Accelerometer sensors are commonly in use to measure motion. Alternatively, motion may be derived from successive camera images as motion vectors. It should be noted, however, that motion is not the same as 'physical effort'. The accelerometer of a wrist worn smartwatch device will notice very different signals for running and biking activity types while the 'physical effort' (e.g. the impact on heart rate) might be similar. Still, for a single activity, it can be expected that 'more motion' indicates 'more effort'.

BRIEF DESCRIPTION OF THE DRAWINGS

These and other aspects of the invention will be apparent from and elucidated with reference to the embodiment(s) described hereinafter. In the following drawings

  Fig. 1 shows a schematic diagram of a first embodiment of a stress detection system and device according to the present invention;

Fig. 2 shows a flow chart of an embodiment of a stress detection method according to the present invention;

Fig. 3 shows a schematic diagram of second embodiment of a stress detection system and device according to the present invention;

Fig. 4 shows a schematic diagram of a third embodiment of a stress detection device according to the present invention;

Figs. 5 to 8 show diagrams of various signals illustrating the detection of mental heart rate with the detection of posture changes; and

Fig. 9 shows a flow chart of another embodiment of a stress detection method according to the present invention.

DETAILED DESCRIPTION OF THE EMBODIMENTS

Heart rate provides the blood and energy transport through the body. A number of life systems that influence the heart rate. First, there is a basal heart rate that appears when someone is in a resting period. The basal heart rate ensures a blood stream sufficient to support energy for basic life systems such as breathing, digestion, heart beating itself, temperature control, etc.

The heart rate increases when people start physical activity up to some level above the basal heart rate where the total increase has some relation with the amount of work executed. This applies to walking, running, carrying, etc.

Mental stress can also cause increases in heart rate. This can be seen as part of body preparation for fight or flight reactions. Hereby, a 'mental stress event' is defined as event experienced by a human, where the event causes a deep unhappy or unsatisfying feeling. Well known tests from psychological research are 'Tone Avoidance Test', 'Sing a Song Stress Test', 'Ravens Progressive Matrices' and 'Paced Auditory Serial Addition Test'.

These heart rate dependencies can be modelled as:

$$HR(t) = HR_{Basal}(t) + HR_{PhysicalActivity}(t) + HR_{MentalStress}(t)$$

Thus, the measured heart rate $HR(t)$ is a combination of three components (or fractions): the basal heart rate component $HR_{Basal}(t)$, the activity heart rate component $HR_{PhysicalActivity}(t)$ and the mental stress heart rate component $HR_{MentalStress}(t)$. This model can be rewritten as:

$$HR_{MentalStress}(t) = HR(t) - HR_{Basal}(t) - HR_{PhysicalActivity}(t)$$

The rewritten model will be applied to determine the mental stress heart rate component $HR_{MentalStress}(t)$.

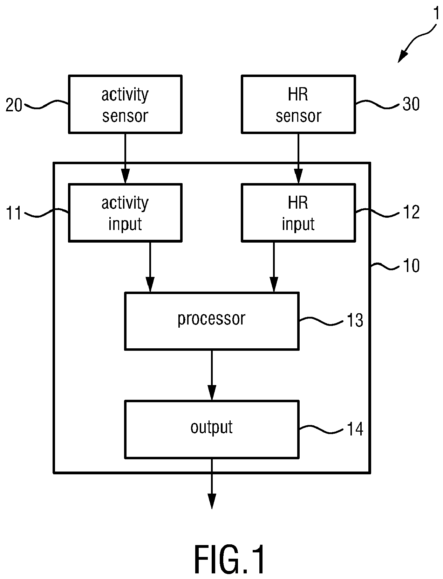

Fig. 1 shows a schematic diagram of a first embodiment of a stress detection system 1 and a stress detection device 10 according to the present invention. The system 1 comprises an activity sensor 20 for acquiring activity information related to activity of the person, a heart rate sensor 30 for acquiring heart rate information indicating or allowing to compute the current heart rate of the person, and the stress detection device 10 for detecting mental stress of a person based on the acquired activity information and the acquired heart rate information.

In an embodiment the activity sensor 20 is mounted on the person's body, e.g. on a wrist, arm, leg or torso, and may include one or more of an accelerometer, a camera, an electrodermal activity sensor, a gyrometer, and a temperature sensor. In other embodiments a device remote from the person may be used as activity sensor 20, such as a remote camera that looks at the person from a distance, e.g. at one or several meters, to detect the person's activity. The activity sensor 20 is generally configured to detect motion allowing to recognize activity of the person and, optionally the strength and/or type of activity.

In an embodiment the heart rate sensor 30 is mounted on the person's body, e.g. on a wrist, arm, leg or torso, and may include one or more of a photoplethysmography sensor, a pulse oximetry sensor, a camera, an ECG sensor, a wristband pressure sensor, and a wristband tension sensor. In other embodiments a device remote from the person may be used as heart rate sensor 30, such as a remote camera using remote photoplethysmography (remote PPG) technology, that looks at the person from a distance, e.g. at one or several meters and evaluates radiation transmitted through or reflected from skin to detect minute light absorption changes in the skin caused by the pulsating blood volume, i.e. by periodic color changes of the human skin induced by the blood volume pulse. This technology is widely known and e.g. described in Verkruijsse et al., "Remote plethysmographic imaging using ambient light", Optics Express, 16(26), 22 December 2008, pp. 21434-21445.

The stress detection device 10 may be implemented in hard- and/or software. For instance, the device 10 may be implemented as appropriately programmed computer or processor. Depending on the application, the device 20 may e.g. be a computer or a workstation or a mobile user device, such as a smartphone, laptop, tablet, smartwatch, etc. For instance, in a practical implementation all elements of the system, including the sensors 20, 30 and the device 10, may be part of a smartwatch or another wearable. In another practical implementation only the sensors 20, 30 are part of such a smartwatch or another wearable, while the device 10 is implemented on a separate device, such as a computer, laptop or smartphone, and a wireless connection (e.g. via Bluetooth or WiFi) is used for data transmission from the sensors 20, 30 to the device 10.

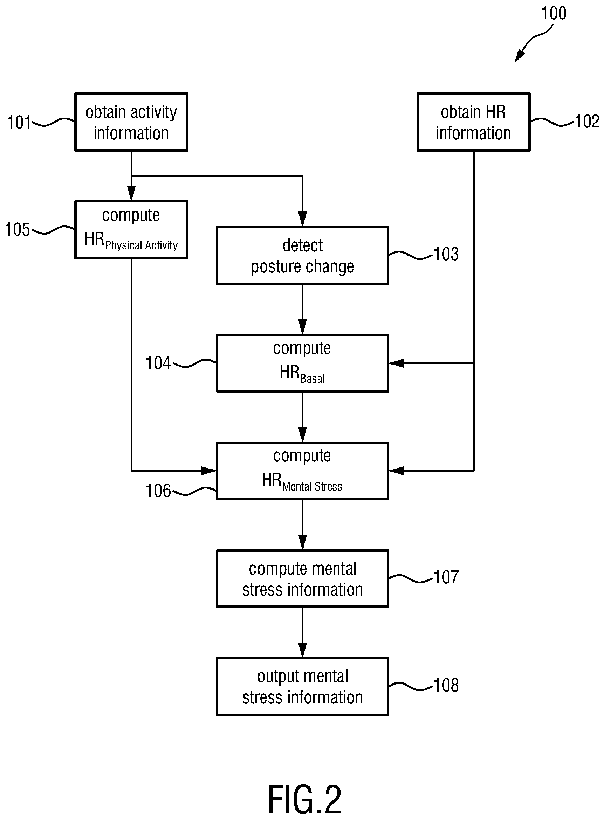

The stress detection device 10 generally comprises an activity input 11, a heart rate input 12, a processing unit 13 and an output 14. Fig. 2 shows a flow chart of a stress detection method 100 that can be performed by the stress detection device.

The activity input 11 obtains (i.e., receives or retrieves; step 101) activity information related to activity of the person, preferably via a direct connection with the activity sensor 20 (or a device including said sensor) or via another entity, such as a memory or a preprocessing unit. The activity information may e.g. comprise one or more of activity count information, action type information and accelerometer information. The activity input 11 may be configured as a conventional wired or wireless data interface, such as a Bluetooth, WiFi or LAN interface.

The heart rate input 12 obtains (i.e., receives or retrieves; step 102) heart rate information indicating or allowing to compute the current heart rate of the person. The heart rate information is preferably obtained via a direct connection with the heart rate sensor 30 (or a device including said sensor) or via another entity, such as a memory or a preprocessing unit. The heart rate information may e.g. comprise a pulse signal or a photoplethysmography (PPG) signal that allows to compute the heart rate, or it may a signal that directly indicates the heart rate. For instance, a heart rate signal (heart rate value over time) may be computed from the obtained heart rate information or may be included in the obtained heart rate information. The heart rate input 12 may be configured as a conventional wired or wireless data interface, such as a Bluetooth, WiFi or LAN interface.

The processor 13 processes (steps 103-107) the obtained activity information and the obtained heart rate information and computes mental stress information related to mental stress of the person. In particular, in a first step 103 it detects a posture change (e.g. from sitting or lying to standing, or from standing to sitting or lying) of the person from the obtained activity information. The result of this posture change detection is then taken into account in a subsequent second step 104 in which a basal heart rate component is computed from the obtained heart rate information. For instance, the basal heart rate component may be computed from the obtained heart rate information under consideration of the result of the posture change detection by computing the basal heart rate component from the obtained heart rate component and adapting the computed heart rate component if a posture change is detected. If no posture change is detected, the computed heart rate component will not be adapted. In another embodiment the computation of the basal heart rate component may be adapted if a posture change is detected, in particular by taking a different computation method and/or adapting one or more parameters used in the computation if a posture change is detected compared when no posture change is detected.

Before or thereafter or in parallel, the processor computes an activity heart rate component from the obtained activity information in step 105.

Subsequently, in step 106 the processor 13 computes a mental stress heart rate component by subtracting, from the current heart rate of the person included in or derived from the obtained heart rate information, the computed activity heart rate component and the computed basal heart rate component. Finally, in step 107 it computes mental stress information related to mental stress of the person from the mental stress heart rate component.

The output 14 outputs (step 108) the computed mental stress information. For instance, it may output this information visually on a display or audibly via a loudspeaker, or it may provide this information to another entity for further processing or rendering. The mental stress information may include information indicating presence or absence of mental stress and/or the level/strength of mental stress of the person.

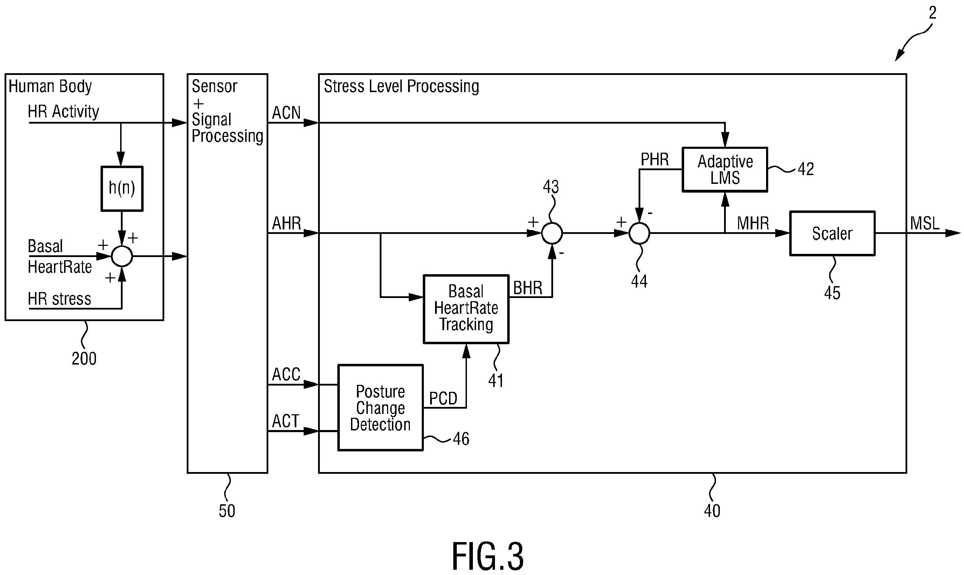

Fig. 3 shows a schematic diagram of second embodiment of a stress detection system 2 and a stress detection device 40 according to the present invention. In addition to the elements of the system 2 a human body model 200 is shown in this figure for illustration.

The human body model 200 illustrates the above-mentioned three components of the measured heart rate $HR(t)$ and a term h(n) that models the human body response from physical activity to heart rate. For instance, when a human starts some physical activity, this will influence the heart rate. The effect is mostly not immediate but with some delay at the start and some delay when the activity ends. The time behavior of these delayed effects can be modelled with the term h(n).

The activity sensor 20 and the heart rate sensor 30 shown in Fig. 1 are included in the sensor and signal processing module 50 that may be implemented as wrist-based activity sensor module. This module 50 may e.g. includes a PPG sensor (as heart rate sensor) and an accelerometer sensor (as activity sensor). Further, in this embodiment the sensor signals acquired by the sensors are preprocessed to deliver higher abstraction level signals including at least a heart rate AHR (which may be an estimate metric of the person's heart rate, such as the instantaneous heart rate or the average heart rate) and an activity count metric ACN and, optionally, an activity type ACT, i.e. this preprocessing extracts the higher abstraction level signals from raw sensor data. Further, the raw accelerometer signal ACC may be provided by the module 50.

During execution of a specific activity type by the user, the heart rate changes due to activity. The change with respect to the basal level is generally (linear) dependent on activity count. The actual relation will be different depending on the type of activity (sitting, walking, running, ...). As human beings change their activity patterns, it can be expected that there is a continuous variation of basal heart rate and that the relation between action count and heart rate increases due to physical activity. But on short time, there will still be the 'linear' relation between activity and heart rate increase with respect to basal level. The adaptive filter updates it's FIR coefficients, for variations in the relation between physical activity and physical heart rate due to variations in activity type, in such a way, that physical activity suppression in the adaptive filter output becomes optimal for the type of activity executed at the moment.

The mental stress detection algorithm applied according to the present invention produces estimates for the three different heart rate components of the above-described heart rate model separately.

The module 50 delivers an AHR metric as an estimate for the current heart rate. In this embodiment the basal heart rate tracking module 41 estimates $HR_{Basal}$, i.e. the basal heart rate component BHR, as the lower envelope of the average heart rate signal AHR. For instance, BHR tracks AHR with different time constants depending on which of AHR or BHR is having the largest value, as will be explained in more detail below.

The module 50 further delivers an ACN metric with a value that increases with the amount of 'physical activity'. This assumption is valid as long as the user keeps on going with similar activity sitting, walking, running, thinking, etc. For such situations, a linear dependency is assumed between $HR_{PhysicalActivity}(t)$ and ACN. An Adaptive Least Minimum Squares Adaptive Filter 42 is used to determine contributions of ACN in the mental heart rate contribution MHR so that physical contributions cancel out in the mental heart rate contribution MHR. In other words, the adaptive filter 42 removes content correlated to ACN from the mental heart rate contribution by correlating a series of samples of ACN with MHR such that the filter output PHR (physical heart rate) mimics the activity heart rate contribution due to activity.

By subtracting both BHR and PHR from the AHR by a common or separate subtraction module(s) 43, 44, the mental stress heart rate contribution MHR is obtained. A scaler module 45 (e.g. applying a log scale) converts the mental stress heart rate contribution MHR into a presentable value representing the mental stress information, such as the mental stress level MSL.

It is a known effect that heart rate depends on people's posture. Even when the user stands absolutely still, a 10 BPM variation between lying, sitting and standing posture can be expected. This affects the determination of the mental stress information because standing up is little visible in the ACN activity signal but causes a relative big change in heart rate. This problem is solved according to the present invention by adding detecting a posture change detection module 46 that detects if there is a posture change or not. The output PCD (posture change detection) is provided to the basal heart rate tracking module 41. If no posture change is detected, the basal heart rate and its computation will not change. If a posture change is detected, the basal heart rate and/or its computation are changed by the basal heart rate tracking module 41.

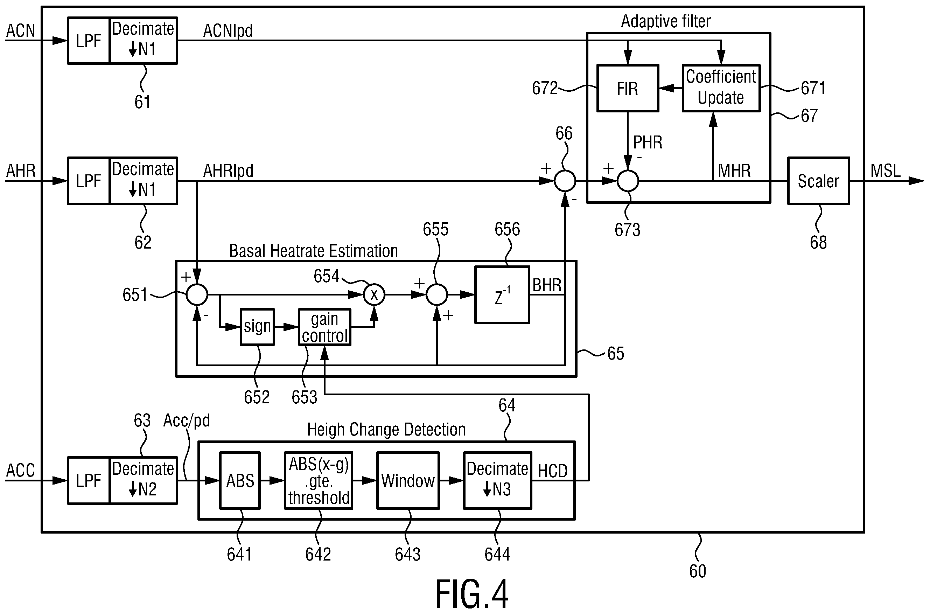

Fig. 4 shows a schematic diagram of a third embodiment of a stress detection device 60 according to the present invention. In this embodiment, AHR, ACN and ACC are used as inputs. Their signal sample rate may be higher than the minimum required in view of signal bandwidth. The input signals AHR and ACN may e.g. have a sample rate 1 Hz (N1 = 1). The ACC signal may be sampled at 128 Hz (N2 = 4) or 32 Hz (N2 = 1) to bring both to 32 Hz. Low pass filters (LPF) 61, 62, 63 are used to reduce noise and as preparation for a decimation stage. The LPFs thus prevent aliasing when decimation of the signals to a lower sample rate. The LPFs 61, 62, 63 may be designed as Hanning window, with nLPF taps and a DC gain = 1. The output signals of the LPFs 61, 62, 63 are designate as ACNlpd, AHRlpd and ACClpd, meaning that they are low-pass filtered and decimated.

For posture change detection a height change detection (HCD) module 64 is provided in this embodiment. An absolute module (ABS) 641 calculates the absolute value of the accelerometer value, e.g. abs(ax, ay, az) = sqrt(ax$^2$ + ay$^2$ + az$^2$). A posture change detection unit (ABS(x-g)) 642 compares the absolute value of the accelerometer readings against gravity 1 g. If the difference is large, then the person might be standing up or sitting down, which is interpreted as height change or, more general, posture change. After posture change detection, a window timer is started by a window unit 643. The window signal is decimated to 1 Hz by a decimation factor N3 = 32 in a decimation unit 644.

When no posture changes are detected, the basal heart rate estimation module 65 follows rises in heart rate slowly (large time constant) while decreases are tracked much faster (smaller time constant). In this way, the 'lower envelope' of the heart rate signal can be followed, which is called the basal heart rate. When the window signal is active (i.e. if a posture change has detected), the basal heart rate estimation module 65 is controlled to track the heart rate signal very fast, e.g. with a small time constant. In this way, the basal heart rate adapts very fast to the value that matches the new posture.

In particular, a subtraction unit 651 takes the difference of the actual heart rate AHRlpd and the current basal heart rate. A sign unit 652 determines the sign of the result of this subtraction. Depending on the sign and the HCD posture detected signal provided by the HCD module 64, the gain control unit 653 proposes different multiplication constants for the lower envelope tracking system. A multiplier 654 multiplies the difference results from the subtraction unit 651 with the multiplication constant proposed by the gain control unit 653. The result is integrated, e.g. added by an addition unit 655 to the current basal heart rate value and subjected to a delay by a delay unit 656 applying a single cycle delay.

In other words, the BHR estimation module 65 forms a first order IIR low pass filter with variable controlled time constants. Basal heart rate tracking takes the difference between the current basal heart rate value BHR and the actual (filtered, down sampled) heart rate value AHR. The next clock, the new BHR is calculated taking in account the value of the difference, the sign of this difference and the window signal (HCD).

The output BHR from the BHR estimation module 65 is subtracted from the actual heart rate AHRlpd by subtraction unit 66. The resulting difference is then subjected to adaptive filtering. Finally, the adaptive filter 67 removes content correlated to ACN from the mental heart rate MHR signal. For this purpose, an adaptive filter unit 672 (e.g. an adaptive LMS (Least-Mean-Squares) filter) correlates a series of samples ACN with MHR. The FIR (finite impulse response) coefficients of the adaptive filter unit 672 are updated or adjusted in an update unit 671 in such a way that the correlation values decrease in the next step. The FIR coefficients are used to filter the ACN signal such that the filter output PHR mimics the heart rate contribution due to activity.

A user's heart rate does not respond immediately to activity changes. A delay of seconds is expected so that the physical contribution to heart rate can be estimated with a linear filter operation FIR:

$$HR_{PhysicalActivity}(t) \sim= \text{FIR(ACN)}$$

In terms of metrics it can be written:

$$MHR(n) = AHR(n) - BHR(n) - \sum_{m=1}^{nFIR} FIR\,(m) * ACN(n-m)$$

where *n* is the index to time sample number, *FIR* are coefficients of the linear filter, and *nFIR* is the number of taps of the linear filter. The impulse response of the system has a duration of *nFIR* samples, so that any *ACN* data sample affects heart rate for a duration of *nFIR* samples. As mentioned above, the filter coefficients of *FIR*() are not constant and will vary depending on user activities and environmental influences. An adaptive filter is thus used that continuously estimates and adapts the coefficients of *FIR*().

It is assumed that signals $HR_{MentalStress}(t)$ and $HR_{PhysicalActivity}(t)$ are uncorrelated. The adaptive LMS filter 671 helps to continuously estimate the *FIR* coefficients 672. The filter determines contributions of *ACN* in *MHR* (correlation, inner product) and adapts the filter coefficients such that the correlation result goes to minimal in a minimum square sense. As a result, the physical contributions cancel out in the mental heart rate signal. New *FIR* coefficients are adjusted every sample period. Hence, a *FIR(n, 1:nFIR)* is assumed where n is the actual sample index and *1:nFIR* are the filter coefficients.

Then, at initialization it is set *FIR* (1,1: *nFIR*) = [000 ... 0] (*nFIRzeros*). For the next samples it is calculated for m = 1: *nFIR*

$$FIR(n+1, m) = FIR(n, m) + kAdaptive * MHR(n) * ACN(n-m).$$

*kAdaptive* is a constant for tuning convergence and tracking speed and stability of the filter.

By subtracting PHR from the difference between AHR and BHR in a subtraction unit 673, MHR is obtained, which is converted by the scaler 68 into the mental stress information MSL.

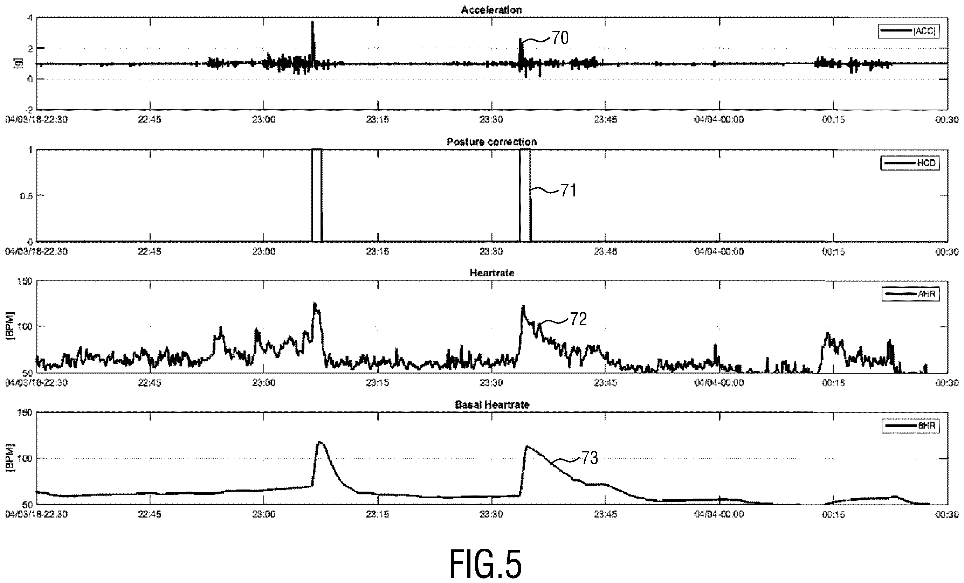

Fig. 5 shows |ACC| 70 and posture correction signal HCD 71 active in periods 23:07 to 23:08 and 23:35 to 23:36. Further, the heart rate signal 72 and the basal heart rate signal 73 are shown. Fast tracking mode with kfast is active in the periods where HCD is active. Slow tracking with krise is active from 00:13 to 00:23. Normal tracking with kfall is active from 00:23 to 00:26.

Figure 30135_pcor_HRcomponents.png shows the activity signal ACN and the reconstructed physical heart rate PHR.

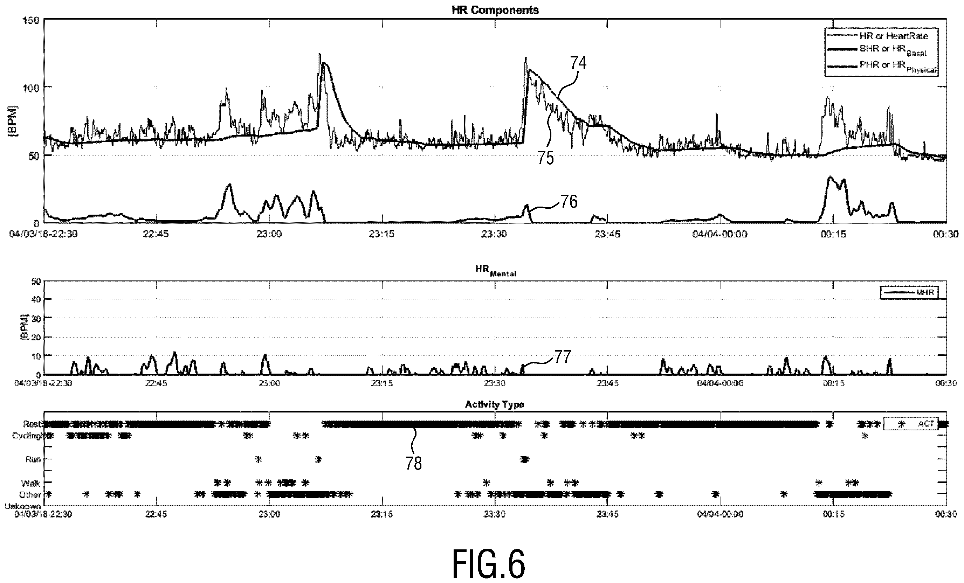

Fig. 6 gives impressions about relative signal amplitudes of the HR signal 74, BHR signal 75, PHR signal 76 and MHR signal 77 and shows the ACT activity type signal 78.

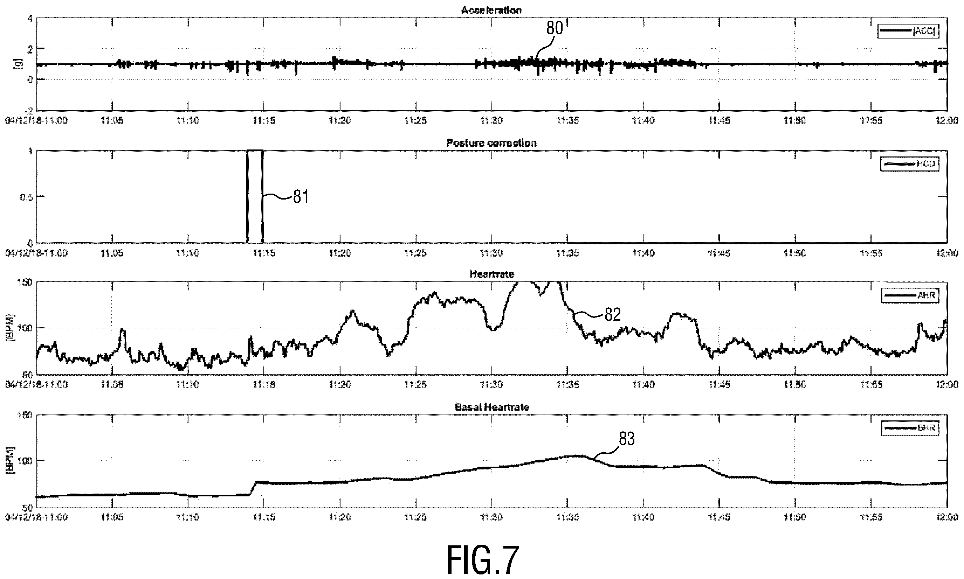

Fig. 7 shows |ACC| 80 and posture correction signal HCD 81 active and fast tracking between 11:14 and 11:15. Slow tracking is active from 11:25 to 11:37 and normal tracking is active from 11:37 to 11:38 and 11:43 to 11:50. Further, the heart rate signal 82 and the basal heart rate signal 83 are shown.

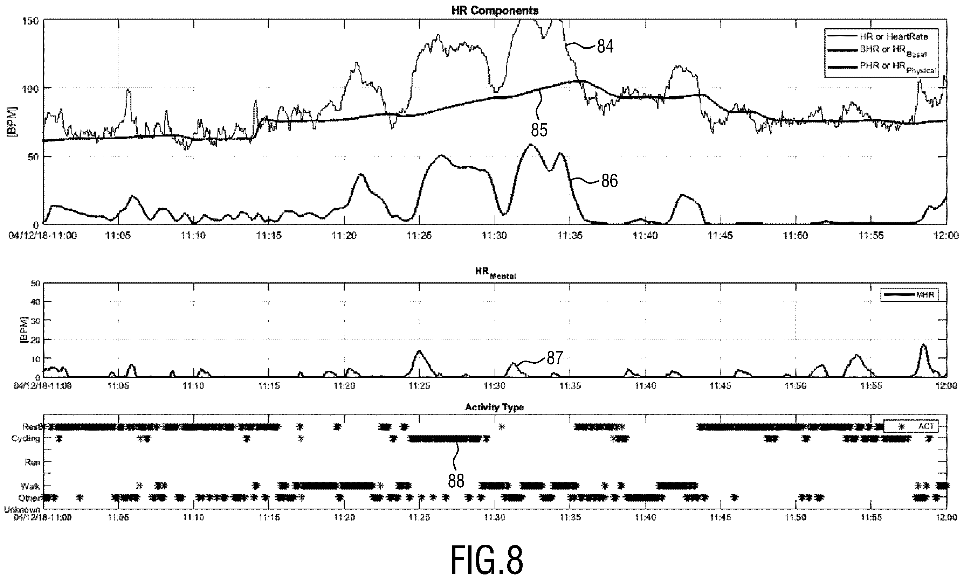

Fig. 8 gives impressions about relative signal amplitudes of the HR signal 84, BHR signal 85, PHR signal 86 and MHR signal 87 and shows the ACT activity type signal 88.

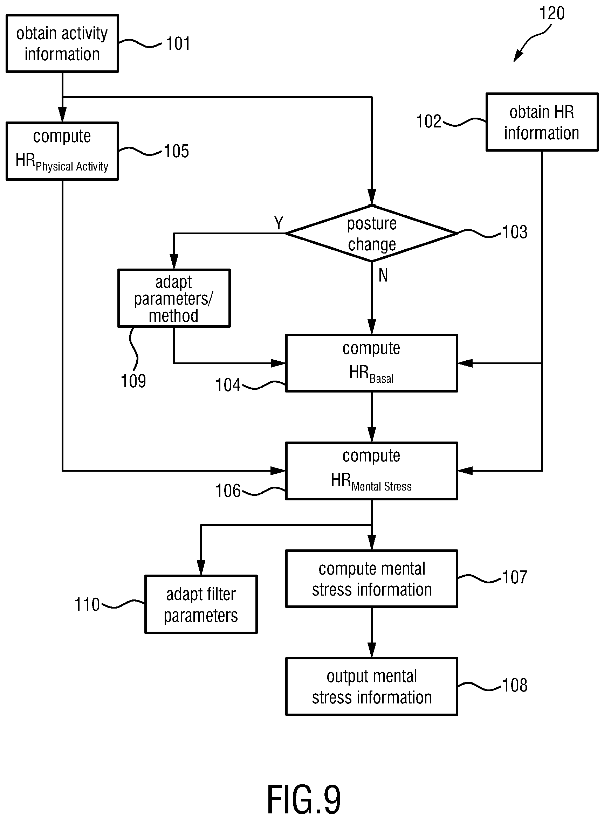

Fig. 9 shows a flow chart of another embodiment of a stress detection method 120 according to the present invention. It contains partly the same steps as the embodiment of the method 100 illustrated in Fig. 2, which are indicated by the same reference signs and will not be explained above. Different from the method 100, the method 120 includes a step 109 of adapting parameters and/or a computation method for computing the basal heart rate in step 105 if a posture change is detected. This step 109 is omitted if no posture change is detected.

Based on the basal heart rate computed in step 105, the obtained heart rate information, and the computed activity heart rate component the mental stress heart rate component is computed in step 106.

In addition to computing the mental stress information in step 107, the computed mental stress heart rate component may further be used in step 110 to adapt filter parameters of the adaptive filter (42 in Fig. 3, 67 in Fig. 4) that may be used to cancel out activity contributions from the heart rate signal as explained above. The adaptive filter may be realized by steps 105 and 110, wherein step 105 includes filtering by an FIR filter that produces the activity heart rate contribution from the activity information and step 110 updates (i.e. adapts) the FIR coefficients using a current value of adaptive filter coefficients, activity information and mental heart rate component.

As explained above, heart rate depends on posture. For detecting posture change a superfast tracking of the BHR signal after height change detection may be implemented. The expression 'superfast tracking' is used here to distinguish it from fast tracking described above.

A simple method to detect height changes is to monitor the norm of the accelerometer signals. If a user stays still, the accelerometer reading is close to gravity g. As soon as the user changes posture, the accelerometer reading is expected to become more different from gravity. This detection works when the user stands up, but also when he sits down. Statistical analysis of results from this simple posture effect reduction system showed a clear improvement compared with the original algorithm.

Any height change detection event re-triggers a window with a certain duration. During this window, the basal heart rate estimation function will operate in fast follow mode. This detection can be described with the following pseudo-code:

```
# During initialization it sets:

BHR(1) = 60

# Then for all samples in the future

If BHR(n) > AHR(n)                        % AHR is lower

BHR(n+1) = BHR(n) + kfall * ( AHR(n) – BHR(n) )  % -> Fast tracking

Else if HCD_window                        % Posture change detection window

BHR(n+1) = BHR(n) + kfast * ( AHR(n) – BHR(n) )  % -> SuperFast tracking

Else                                      % AHR is higher

BHR(n+1) = BHR(n) + krise * ( AHR(n) – BHR(n) )  % -> Slow tracking

End
```

In other embodiments a larger number of constants may be used to address more different conditions, posture changes and activity changes. For example, ((ACT==Sitting) & (HR > BHR)), ((ACT==Sitting) & posture change & (BHR > HR)), ((previous ACT==Sitting) -> (ACT == Walking)).

The output metric ARL MSL is a scaled version of the MHR metric. Scaling may be performed according to the formula:

$$ARL = min( R\text{-}1, floor( R/\log2(51)*(log2(floor(2^M*(1+kscale*MHR)))\text{-}M) ) )$$

with number of scale levels R = 1000, scaling constant *kscale* = 1 (which can be modified to tune the stress level results), accuracy and *M* = 10. This results in:

| MHR | ARL |
|-----|-----|
| 0.0 | 000 |
| 1.5 | 233 |

(continued)

| MHR | ARL |
|---|---|
| 5.00 | 455 |
| 10.00 | 609 |
| 20.00 | 774 |
| 50.00 | 999 |
| Above 50.00 | 999 |

Heart rate changes due to posture changes can be considered as different basal metabolic heart rates depending on the posture. According to the present invention, posture changes are detected from accelerometer signals and a posture change detection changes the basal heart rate by a tracking mechanism by application of a short time constant during a time window starting after posture change detection.

There are different simple and complex methods available to detect posture changes from accelerometer data.

According to a particular embodiment, in rest a 3-axis accelerometer measures 1 g $\sim$= 9.81 m/s$^2$. When the person stands up from sitting, this value will temporary increase above 1 g. The basal heart rate superfast tracking window may then be triggered when

$$Trigg1 = ( \, |acc| - 9.81 \, ) > Threshold1.$$

According to another embodiment posture changes from standing to sitting can be detected by

$$Trigg2 = | \, |acc| - 9.81 \, | > Threshold2.$$

This embodiment does not distinguish between stand up or sit down. Therefore, it might enable faster response of the mental stress detection after sit down.

When the wearable device is worn on the wrist, most people have their hand pointing down when standing and pointing horizontal when sitting. For instance, the orientation of the accelerometer depends on the posture. This alternative implementation uses orientation information to detect posture changes and improves the accuracy of the mental stress detection.

A 'Activity Type' metric can take values like 'Rest', 'Running', 'Cycling', 'Walking', 'Other'. Changes in this metric value can also indicate posture changes.

In an embodiment a new basal heart rate value is estimated by taking the difference of actual heart rate and current basal heart rate, multiplying it with a constant, and adding it to the current basal heart rate value can be seen as a first order low pass IIR filter. Such a filter can be characterized by a low pass bandwidth and/or time constants. The filter time constants depend on the filter sample frequency and the multiplication factor. Different tracking time constants are realized by different multipliers that are selected at conditions: PostureChangedWindow, HR>BHR, HR<BHR. The latter two conditions create the 'lower envelope tracking' behavior.

In analog low pass filter terms, the time constants for these different conditions may be 1/(Fs*kfast), 1/(Fs*krise) and 1/(Fs*kfall). In general it holds: kfast>> kfall >> krise. The values of the constants may be chosen by inspection of recorded signals of numerous test candidates. In other embodiments, optimization on a person to person base may be made. In an exemplary implementation Fs = 1 Hz and kfast = 0.1, kfall = 0.01 and krise = 0.001. Other values may be used in other embodiments.

In a practical realization of the disclosed stress detection system the sensor module is a wrist worn device that embeds PPG and motion sensors and a host processor device. Continuous data streams from both sensors feed software that runs on the host and extracts a number of high level metrics characterizing the physical state of the wearer of the device. These include AHR, an estimate for heart rate, and ACN, an action count that holds a measure for the amount of motion ($\sim$= physical activity) that the user applies.

A new mental stress metric is added. The calculation of the mental stress metric requires AHR, ACN and ACC as inputs. Output from the algorithm is logged as ARL metric. For instance, there is a visual indication on a 3-color LED.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Stress detection device (10, 40, 60) for detecting mental stress of a person, the device comprising:

    an activity input (11) configured to obtain activity information related to activity of the person;
    a heart rate input (12) configured to obtain heart rate information indicating or allowing to compute the current heart rate of the person;
    a processing unit (13) configured to

    - detect a posture change of the person from the obtained activity information,
    - compute a basal heart rate component from the obtained heart rate information under consideration of the result of the posture change detection,
    - compute an activity heart rate component from the obtained activity information,
    - compute a mental stress heart rate component by subtracting, from the current heart rate of the person, the computed activity heart rate component and the computed basal heart rate component, and
    - compute mental stress information related to mental stress of the person from the mental stress heart rate component; and

    an output (14) configured to output the computed mental stress information.

2. Device as claimed in claim 1,
    wherein the processing unit (13) is configured to compute the basal heart rate component from the obtained heart rate information under consideration of the result of the posture change detection by

    - computing the basal heart rate component from the obtained heart rate component and adapting the computed basal heart rate component if a posture change is detected, and/or
    - adapting the computation of the basal heart rate component if a posture change is detected, in particular by taking a different computation method and/or adapting one or more parameters used in the computation.

3. Device as claimed in claim 1,
    wherein the processing unit (13) is configured to compute the basal heart rate component as lower envelope of a heart rate signal of the obtained heart rate information.

4. Device as claimed in claim 3,
    wherein the processing unit (13) is configured to adapt the computation of the basal heart rate component if a posture change is detected by increasing, during a time window after detection of a posture change, the speed by which the basal heart rate follows the heart rate signal.

5. Device as claimed in claim 3,
    wherein the processing unit (13) is configured to compute the basal heart rate component by taking the difference between the current value of the heart rate signal and the current value of the basal heart rate, multiplying the difference with a multiplication constant and obtaining a new value for basal heart rate by integration of the result.

6. Device as claimed in claim 5,
    wherein the processing unit (13) is configured to use

    - a first multiplication constant if the current value of the basal heart rate is larger than the current value of the heart rate signal,
- a second multiplication constant if the current value of the basal heart rate is smaller than the current value of the heart rate signal, and
- a third multiplication constant if a posture detection is detected,

wherein the second multiplication constant is smaller than the first multiplication constant and the first multiplication constant is smaller than the third multiplication constant.

7. Device as claimed in claim 1,
wherein the processing unit (13) is configured to compute the basal heart rate component from the obtained heart rate component and adapt the computed basal heart rate component if a posture change is detected by increasing the computed basal heart rate component if the posture changes from a sitting or lying posture to a standing posture and by decreasing the computed basal heart rate component if the posture changes from a standing posture to a sitting or lying posture.

8. Device as claimed in any one of the preceding claims,
wherein the processing unit (13) is configured to detect a posture change of the person from the obtained activity information by detecting a change of a measured acceleration above an acceleration threshold and/or by detecting a change of the orientation of a sensor that is configured to acquire the activity information.

9. Device as claimed in any one of the preceding claims,
wherein the processing unit (13) is configured to detect a posture change of the person from the obtained activity information by detecting changes in an activity type metric indicating the type of activity of the person determined from the obtained activity information.

10. Device as claimed in any one of the preceding claims,
wherein the activity information comprises one or more of activity count information, action type information and accelerometer information.

11. Device as claimed in any one of the preceding claims,
where the processing unit (13) is configured to compute the activity heart rate component from the obtained activity information by minimizing the correlation between the activity heart rate component and the mental stress heart rate component.

12. Stress detection system (1, 2) for detecting mental stress of a person, the system comprising:

- an activity sensor (20) configured to acquire activity information related to activity of the person;
- a heart rate sensor (30) configured to acquire heart rate information indicating or allowing to compute the current heart rate of the person; and
- a stress detection device (10, 40, 60) as defined in any one of the preceding claims for detecting mental stress of a person based on the acquired activity information and the acquired heart rate information.

13. System as claimed in claim 12,
wherein the activity sensor (20) includes one or more of an accelerometer, a body-mounted camera, a remote camera, an electrodermal activity sensor, a gyrometer, and a temperature sensor, and/or
wherein the heart rate sensor (30) includes one or more of a photoplethysmography sensor, a pulse oximetry sensor, a body-mounted camera, a remote camera, an ECG sensor, a wristband pressure sensor, and a wristband tension sensor.

14. Stress detection method for detecting mental stress of a person, the method comprising:

- obtaining activity information related to activity of the person;
- obtaining heart rate information indicating or allowing to compute the current heart rate of the person;
- detecting a posture change of the person from the obtained activity information;
- computing a basal heart rate component from the obtained heart rate information under consideration of the result of the posture change detection;
- computing an activity heart rate component from the obtained activity information;
- computing a mental stress heart rate contribution by subtracting, from the current heart rate of the person, the computed activity heart rate contribution and the computed basal heart rate component;
- computing mental stress information related to mental stress of the person from the mental stress heart rate component; and
- outputting the computed mental stress information.

15. Computer program comprising program code means for causing a computer to carry out the steps of the method as claimed in claim 14 when said computer program is carried out on the computer.

1

| 20 | activity<br>sensor | HR<br>sensor | 30 |

| 11 | activity<br>input | HR<br>input | 12 |

10

| processor | 13 |

| output | 14 |

# FIG.1

100

```
101 ──  obtain activity                    obtain HR      ── 102
           information                    information

105 ──  compute
        HR_Physical Activity          detect
                                   posture change  ── 103

                                     compute
                        104 ──       HR_Basal

                                     compute
                        106 ──       HR_Mental Stress

                                 compute mental       ── 107
                                 stress information

                                 output mental        ── 108
                                 stress information
```

# FIG.2

2

Stress Level Processing

Scaler — 45

MSL

Adaptive LMS — 42

MHR

PHR

− +

44

43

+ −

BHR

Basal HeartRate Tracking — 41

PCD

Posture Change Detection — 46

40

Sensor + Signal Processing

ACN

AHR

ACC

ACT

50

Human Body

HR Activity

h(n)

+ +

+

Basal HeartRate

HR stress

200

FIG.3

FIG.4

EP 4 011 285 A1

FIG.5

FIG.6

FIG.7

FIG.8

EP 4 011 285 A1

FIG.9

Europäisches
Patentamt

European
Patent Office

Office européen
des brevets

# EUROPEAN SEARCH REPORT

**Application Number**

EP 20 21 2775

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2016/338640 A1 (CHAN ALEXANDER [US] ET AL) 24 November 2016 (2016-11-24) * paragraphs [0024], [0032], [0034], [0036], [0037], [0040], [0043], [0060], [0062], [0063] * ----- | 1-15 | INV. A61B5/024 A61B5/11 A61B5/16 |
| A | US 2015/150516 A1 (TOCHIKUBO OSAMU [JP] ET AL) 4 June 2015 (2015-06-04) * paragraphs [0075], [0079], [0170] - [0175], [0218], [0220], [0222] * ----- | 1-15 | |
| A | US 5 267 568 A (TAKARA ATSUNORI [JP]) 7 December 1993 (1993-12-07) * column 5, lines 42-58 * * column 6, lines 60-63 * ----- | 1-15 | |
| A | EP 2 903 508 A1 (FORSKARPATENT I LINKÖPING AB [SE]; BENOSMANE MOURAD [DZ]) 12 August 2015 (2015-08-12) * paragraphs [0014], [0035] - [0038] * ----- | 1-15 | **TECHNICAL FIELDS SEARCHED (IPC)** A61B |

The present search report has been drawn up for all claims

| Place of search | Date of completion of the search | Examiner |
|---|---|---|
| The Hague | 6 May 2021 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 2775

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2021

| Patent document cited in search report | | Publication date | Patent family member(s) | | Publication date |
|---|---|---|---|---|---|
| US 2016338640 | A1 | 24-11-2016 | US | 2016338640 A1 | 24-11-2016 |
| | | | US | 2018310879 A1 | 01-11-2018 |
| US 2015150516 | A1 | 04-06-2015 | CN | 104602604 A | 06-05-2015 |
| | | | EP | 2893878 A1 | 15-07-2015 |
| | | | JP | 6047346 B2 | 21-12-2016 |
| | | | JP | 2014050451 A | 20-03-2014 |
| | | | US | 2015150516 A1 | 04-06-2015 |
| | | | WO | 2014038594 A1 | 13-03-2014 |
| US 5267568 | A | 07-12-1993 | AU | 640044 B2 | 12-08-1993 |
| | | | BE | 1004497 A5 | 01-12-1992 |
| | | | CH | 685917 A5 | 15-11-1995 |
| | | | DE | 4133608 A1 | 21-05-1992 |
| | | | FR | 2669450 A1 | 22-05-1992 |
| | | | GB | 2251490 A | 08-07-1992 |
| | | | IT | 1258223 B | 21-02-1996 |
| | | | JP | H0558730 B2 | 27-08-1993 |
| | | | JP | H04180730 A | 26-06-1992 |
| | | | KR | 920010272 A | 26-06-1992 |
| | | | US | 5267568 A | 07-12-1993 |
| EP 2903508 | A1 | 12-08-2015 | EP | 2903508 A1 | 12-08-2015 |
| | | | WO | 2014053538 A1 | 10-04-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VERKRUIJSSE et al.** Remote plethysmographic imaging using ambient light. *Optics Express,* 22 December 2008, vol. 16 (26), 21434-21445